## Europäisches Patentamt
## European Patent Office
## Office européen des brevets

(19)

(11) Veröffentlichungsnummer: **0 308 531**
**B1**

(12) **EUROPÄISCHE PATENTSCHRIFT**

(45) Veröffentlichungstag der Patentschrift:
**27.12.90**

(51) Int. Cl.⁵: **G01N 33/00, F28D 7/02**

(21) Anmeldenummer: **87113949.9**

(22) Anmeldetag: **23.09.87**

(54) **Messgaskühleinrichtung.**

(43) Veröffentlichungstag der Anmeldung:
**29.03.89 Patentblatt 89/13**

(45) Bekanntmachung des Hinweises auf die Patenterteilung:
**27.12.90 Patentblatt 90/52**

(84) Benannte Vertragsstaaten:
**AT BE CH DE ES FR GB GR IT LI LU NL SE**

(56) Entgegenhaltungen:
**DE-A- 3 111 415**
**DE-A- 3 117 431**
**FR-A- 2 155 770**
**GB-A- 598 084**
**US-A- 2 218 594**
**US-A- 2 681 797**
**US-A- 3 696 636**
**US-A- 3 861 165**
**US-A- 3 871 444**
**US-A- 4 174 500**

(73) Patentinhaber: **VIA Gesellschaft für Verfahrenstechnik mbH, Heerdter Lohweg 63-71, D-4000 Düsseldorf 11(DE)**

(72) Erfinder: **Schlensker, Herbert Dipl.-Ing., Grundermühlenweg 24, D-5090 Leverkusen 3(DE)**

(74) Vertreter: **Müller, Gerd, Patentanwälte HEMMERICH-MÜLLER-GROSSE-POLLMEIER-MEY-VALENTIN Hammerstrasse 2, D-5900 Siegen 1(DE)**

ACTORUM AG

**Beschreibung**

Die Erfindung betrifft eine Meßgaskühleinrichtung, bestehend aus einem einen Teil des Kältemittelkreislaufs eines Kühlaggregats bildenden Wärmetauscher, der zusammen mit einem Kondensatabscheider in einem nach außen wärmeisolierenten Behälter angeordnet ist, wobei der Wärmetauscher von einem in dem Behälter im wesentlichen senkrecht angeordneten Rohrstück gebildet wird, an dessen unterem Ende eine Rohrleitung mit einem Rohrdurchmesser, der kleiner als der des Rohrstücks ist, angeschlossen ist, die um den Außenumfang des Rohrstücks wendelförmig in nach oben gerichteter Wendelsteigung gewickelt ist, und wobei das obere Ende des Rohrstücks und das obere Ende der Rohrleitungswendel durch die Wand des Behälters dicht hindurchgeführt sind.

Eine derartige Meßgaskühleinrichtung ist aus der von der Anmelderin stammenden DE 3 111 415 A1 bereits bekannt.

Außerdem ist aus der US-A 3 861 165 ein Wärmetauscher für eine Luftentfeuchtungseinrichtung bekannt, bei dem eine zweite Rohrleitung wendelförmig im Behälter angeordnet ist und deren zwei Enden durch die Wand des Behälters dicht hindurchgeführt sind, und die über im Behälter befindliche Wärmeübertragungsflüssigkeit mit der ersten Rohrleitung im Wärmekontakt steht und vom Kältemittel des Kühlaggregats durchströmt ist.

Die bekannte Meßgaskühleinrichtung arbeitet an sich recht zufriedenstellend, doch hat sie sich bei bestimmten Anwendungsfällen als zu unflexibel bei der Verwendung des Materials für die das Meßgas führenden Bauteile erwiesen. Das üblicherweise verwendete Kältemittel greift nämlich bestimmte Materialien, insbesondere viele Kunststoffe, an und steht außerdem unter einem Betriebsdruck, der meist erheblich höher als der Druck des Meßgases ist: Während das Kältemittel einen Druck in der Grössenordnung von 2...3 bar aufweisen kann, liegt der Druck des Meßgases meist weit unter einem Wert von 1 bar, z.B. zwischen 0,1 und 0,5 bar.

Aufgabe der Erfindung ist es, die bekannte Meßgaskühleinrichtung derart weiterzubilden, daß für solche Teile, die kein Kältemittel führen, eine größere Flexibilität hinsichtlich der benutzten Materialien erreicht wird. Dadurch lassen sich bei Anwendungsfällen, in denen z. B. das Meßgas unter sehr viel niedrigerem Druck (oder unter sehr viel höherem Druck) als der Druck des Kältemittels steht, erhebliche Einsparungen bei den Produktionskosten erreichen. Desweiteren wird der Einsatz von transparenten Materialien (wie Glas oder transparente Kunststoffe) erleichtert, was Vorteile bei der Überwachung und Wartung der Meßgas-Kühleinrichtung haben kann.

Der Einsatz von Glas als Wärmetauscherrohrmaterial ist bei der bekannten Anordnung deshalb problematisch, weil bei rauher Behandlung es schon einmal zum Bruch des Glasrohres kommen kann, was beim Stand der Technik zum ungewünschten Austritt von Kältemittel führen würde.

Außerdem sollte eine Erhöhung des Kühlmittelgrades des Wärmetauschers erreicht und der Kühleffekt des Kühlmittels maximiert werden.

Gelöst wird die Aufgabe dadurch, daß um das Rohrstück eine zweite Rohrleitung wendelförmig gewickelt ist, deren zwei Enden durch die Wand des Behälters dicht hindurchgeführt sind, und die über im Behälter befindliche Wärmeübertragungsflüssigkeit mit der ersten Rohrleitung in Wärmekontakt steht und von Kältemittel des Kühlaggregates durchströmt ist, daß die zweite Rohrleitungswendel in einer zwischen Rohrstückaußenfläche und erster Rohrleitungswendel gebildeten Ringraum angeordnet ist, und daß die zweite Rohrleitungswendel einen ersten äußeren Wendelteil und einen zweiten, dazu koaxialen inneren Wendelteil aufweist, wobei die Wendelteile an ihrem unteren Ende miteinander verbunden sind und an ihrem oberen Ende den Kältemitteleinlaß bzw. den Kältemittelauslaß bilden.

Durch diese Maßnahmen wird erreicht, daß beim Aufbau der Meßgaskühleinrichtung – abgesehen von den Bauteilen, die das Kältemittel führen – eine größere Vielfalt von Materialien eingesetzt werden kann. So könnte als Werkstoff für die das Meßgas führenden Teile anstelle von Edelstahl säurefester (aber nicht kältemitteldichter) Kunststoff verwendet werden, wie PVC (Polyvinylchlorid) oder PVDF (Polyvinylidenfluorid). Ähnliches gilt für den wärmeisolierten Behälter. Dadurch, daß die zweite Rohrleitungswendel in einem zwischen Rohrstückaußenfläche und erster Rohrleitungswendel gebildeten Ringraum angeordnet ist, wird die das Kältemittel führende Kühlwendel in die das Meßgas führende Kühlwendel bzw. Rohrstücke eingeschlossen, so daß besonders kurze Wärmeübertragungswege verwirklicht werden können.

Dies bewirkt den angestrebten möglichst guten Wärmeübertragungswert zwischen dem Kältemittel des Kühlaggregats und der das Meßgas führenden Wärmetauscherwendel, indem die zweite Rohrleitungswendel einen ersten äußeren Wendelteil und einen zweiten, dazu koaxialen inneren Wendelteil aufweist, wobei die Wendelteile an ihrem unteren Ende miteinander verbunden sind und an ihrem oberen Ende den Kältemitteleinlaß bzw. den Kältemittelauslaß bilden, ergibt sich nicht nur eine besonders kurze Bauhöhe für den Wärmetauscherbehälter, sondern dies erhöht auch den Kühleffekt.

Gemäß einer anderen Ausführungsform der Erfindung besitzt der Behälter eine verschließbare Öffnung zum Einfüllen von Wärmeübertragungs- bzw. Wärmespeicherungsflüssigkeit, wobei insbesondere Kochsalzlösung oder ein Gemisch aus Wasser und Ethandiol (Glykol) als derartige Flüssigkeit sich bewährt hat. Der Behälter kann auch einen in die Kühlsole eingetauchten Sensor aufweisen, mit dem beispielsweise ein mikroprozessorgesteuertes Betriebskontrollgerät betreibbar ist.

Die Erfindung wird nachfolgend anhand eines Ausführungsbeispiels näher erläutert, das in den Zeichnungen dargestellt ist.

Es zeigt:

Fig. 1 schematisch den Aufbau einer Meßgaskühleinrichtung;

Fig. 2 einer Axialschnittansicht, teilweise mit versetzt dargestellten Rohranschlüssen, eine besonders günstige Ausführungsform für einen wärmeisolierten Behälter mit Wärmetauscher und Abscheider gemäß den Merkmalen der vorliegenden Erfindung; und

Fig. 3 eine Draufsicht auf den Deckel des Behälters der Fig. 2 zur Erläuterung der verschiedenen Rohranschlüsse.

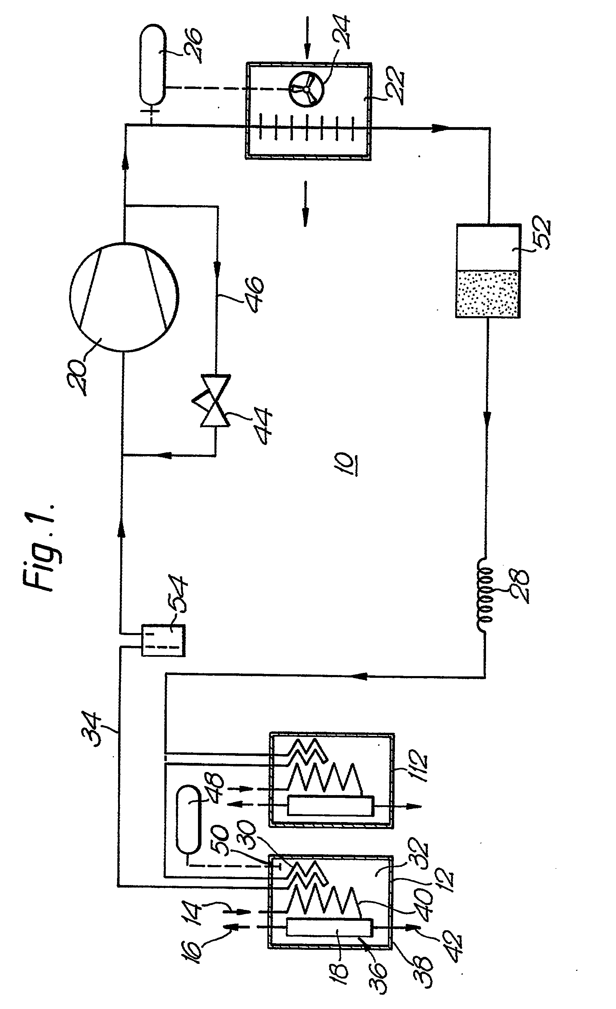

Fig. 1 zeigt eine Meßgaskühleinrichtung 10, die in Gasanalyse-Meßeinrichtungen eingesetzt werden, um feuchtes Meßgas soweit abzukühlen, daß an keiner nachfolgenden Stelle der Gasanalysenmeßeinrichtung der Taupunkt unterschritten wird. Bei bestimmten Meßproblemen ist es erforderlich, den Taupunkt des Meßgases konstant zu halten, um den Einfluß des Wasserdampfes auf das Meßergebnis auszuschalten. Der von der Meßgaskühleinrichtung konstantgehaltene Feuchtigkeitsteil, insbesondere Wasserdampfanteil, läßt sich später bei der Kalibrierung der Gasanalysenmeßeinrichtung berücksichtigen.

Die Meßgaskühleinrichtung besteht in an sich bekannter Weise aus einem (oder mehreren( Wärmetauscher 12, 112, dem das beispielsweise aus einem Abgaskanal stammende. u.U. zeitlich schwankende Feuchtigkeit enthaltend Meßgas 14 zugeführt wird. Innerhalb des Wärmetauschers wirde das Meßgas auf beispielsweise 2°C abgekühlt und dann bei 16 wieder abgegeben. Das dabei anfallende Kondensat des Meßgases wird in einem Abscheidebehälter 18 ausgefällt. Als Kühlmedium wird ein Kältemittel, beispielsweise das mit der Bezeichnung R12, eingesetzt. Ein Kompressor 20 verdichtet das aus dem Wärmetauscher 12 austretende und über einen Dampfdom 54 geführte Kältemittel je nach gewünschten Betriebsbedingungen auf beispielsweise 7 bar. Der Kompressor kann aus einem Druckgehäuse bestehen, in dem sich ein elektromotorisch betriebener Kolbenverdichter befindet.

Im nachfolgenden Kondensator 22 wird das Kältemittel verflüssigt, wobei die Wärmeabfuhr durch einen Ventilator 24 erfolgt, der bei niedriger Umgebungstemperatur von einem kältemitteldruckgesteuerten Ventilatorschalter 26 ausgeschaltet werden kann. Anschließend erfolgt eine Druckreduzierung des Kältemittels durch ein Kapillarrohr 28 beispielsweise ca. 7 bar auf z.B. 2 bar. Das druckreduzierte Kältemittel wird dann in eine Wärmetauscherrohrschlange (30) Verdampfer) eingespritzt, wo es indirekt über Kühlsole 32 Wärmeenergie von Meßgas aufnimmt und durch diesen Vorgang verdampft wird. Der Kompressor 20 saugt das verdampfte Kältemittel über Rohrleitung 34 wieder an. Der Verdampfung 30 befindet sich gemeinsam mit der vom Meßgas durchströmten Wärmetauschereinheit 36 in einem mit Kühlsole gefüllten Behälter 38. Die Wärmetauschereinheit 36 selbst besteht aus einem spiralförmig gewickelten Rohr 40 mit anschließendem Abscheidebehälter 18 und Kondensatablaßrohr 42.

Das Meßgas wird durch Wärmeabgabe an die Kühlsole und diese durch das Kältemittel gekühlt. Alle drei Systeme Meßgas, Kühlsole und Kältemittel sind hermetisch voneinander getrennt. Die Meßgasaustrittstemperatur wird vorzugsweise von einem Heißgasbypassregelventil 44 geregelt, das in Abhängigkeit vom Kältemitteldruck am Verdampferausgang mehr oder weniger Kältemittel vom Ausgang des Kompressors 20 abzieht und dem Kompressoreingang über einen Bypass 46 wieder zuführt.

Da Druck und Temperatur des Kältemittels in unmittelbarem Verhältnis zueinander stehen, gewährleistet dieses Heißgasbypassregelventil in an sich bekannter Weise bei einem eingestellten Verdampferdruck von beispielsweise 2,2 bar auch eine konstante Temperatur des Meßgases innerhalb des Meßgasdurchtrittsbereiches, der für die Vorrichtung vorgesehen ist.

Zur Kontrolle der Temperatur der Sole 32 dient ein mikroprozessorgesteuertes Betriebskontrollgerät, wobei die Temperatur durch eine in die Kühlsole eingetauchte Sensoreinrichtung 50 ermittelt werden kann.

Außerdem ist im Kältemittelkreislauf noch ein Kältemitteltrockner 52 vorhanden.

Umfaßt die Meßgaskühleinrichtung mehr als einen Wärmetauscher 12, beispielsweise für einen zweiten Meßgasweg, kann der Kältemittelkreislauf so geführt sein, daß sich eine Parallelschaltung oder auch eine Hintereinanderschaltung ergibt.

Das anfallende Kondensat wird über ein externes Kondensatgefäß gesammelt, oder von einer automatischen Kondensatablaßeinrichtung abgepumpt.

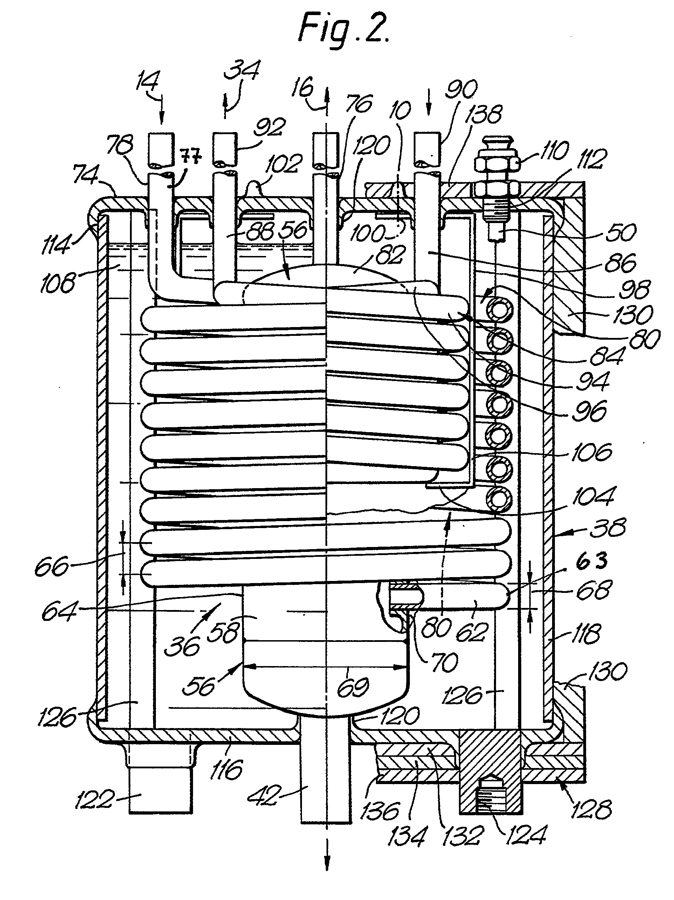

In Fig. 2 ist in einer axialen, teilweise versetzten Schnittansicht ein Wärmetauscher 12 mit einer Wärmetauschereinheit 36 zu erkennen, die von einem Behälter 38 im wesentlichen senkrecht angeordneten Rohrstück 56 gebildet wird, an dessen unterem Ende 58 eine Rohrleitung 62 mit einem Rohrdurchmesser 68, der keiner als der Durchmesser 69 des Rohrstücks 56 ist, angeschlossen ist, siehe Bezugszahl 70. Die Rohrleitung 62 ist um den Außenufmang 64 des Rohrstücks 56 wendelförmig mit nach oben gerichteter Wendelsteigung 66 gewickelt, wobei das obere Ende 82 des Rohrstücks 56 und das obere Ende der Rohrleitungswendel 62 durch die hier einen Deckel bildende Wand 74 des Behälters 38 abgedichtet in Form von Rohranschlüssen 76, 78 hindurchgeführt sind.

Im Gegensatz zum Stand der Technik, wo die Rohrleitung 62 direkt auf die Außenumfangswand 64 des Rohrstücks 56 gewickelt ist, läßt hier diese (erste) Rohrleitungswendel 62 zwischen sich und der Außenfläche 64 des Rohrstücks 56 einen ringförmigen Raum 80 frei, so daß es möglich ist, um das Rohrstück 56 eine zweite Rohrleitung 84 wendelförmig aufzuwickeln, deren zwei Enden 86, 88 durch die (wiederum den Deckel) bildende Wand 74 des Behälters 38 dicht hindurchgeführt sind und Anschlußleitungen 90 bzw. 92 für die Zufuhr bzw. den Abzug von Kältemittel bilden.

Wie zu erkennen ist, bildet die zweite Rohrleitungswendel 84 einen ersten äußeren Wendelteil 94 und einen zweiten, dazu koaxialen inneren Wendelteil 96, die an ihren unteren Enden miteinander verbunden sind und an ihrem jeweiligen oberen Ende 88 bzw. 86 mit dem Kältemittelauslaß 92 bzw. Kältemitteleinlaß 90 in Verbindung stehen. Insbesondere ist das obere Ende der inneren Wendel 96 mit dem Kältemitteleinlaß 90 verbunden. Äußerer Wendelteil 94, innerer Wendelteil 96 sowie Einlaß 90 und Auslaß 92 können einstückig aus Rohrmaterial gefertigt sein.

Die die beiden Rohrleitungswendeln bildende Rohrleitung wird aus Metall hergestellt sein, insbesondere aus Kupfer, da PVC (Polyvinylchlorid) oder PVDF (Polyvinylidenfluorid) nicht kältemitteldicht sind. Es ist zweckmäßig, die mit Kältemittel beaufschlagte zweite Rohrleitung wegen ihrer Flexibilität durch Stützeinrichtung 98 zu umfassen, wobei diese Stützeinrichtungen beispielsweise von drei im Abstand von 120° angeordneten Haltewinkeln gebildet sein können, die ihrerseits beispielsweise mittels Schraubbefestigung (Bezugszahl 100) am Deckel 74 befestigt sein können, wobei

durch Nasen 102 ein abgedichtetes Sackgewinde ausreichender Länge gebildet werden könnte. Die Haltwinkel stützen insbesondere am unteren Ende durch eine Abbiegung 104 die unterste Wendel der zweiten Rohrleitung 84 sowie mit dem Seitenteil 106 die koaxiale Ausrichtung dieser Rohrleitung. Die Stützeinrichtung 98 könnte aber auch die Form eines Zylinders mit geschlossener Wandfläche haben, wodurch sich eine gewisse strömungstechnische Führung der im Behälter 38 eingefüllten Salzsole 108 ergibt, falls z.B. Umwälzpumpeneinrichtungen vorgesehen sind.

Zum Einfüllen der Sole 108 kann eine hier nicht näher dargestellte verschließbare Einfüllöffnung dienen, oder die Sole wird während des Herstellungsprozesses eingefüllt und ist nach Aufsetzen des Deckels 74 auf den Behälter 38 und dessen Verklebung an der Stelle 114, falls auch Behälterwand und Behälterdeckel aus Kunststoff bestehen, dicht eingeschlossen. In ähnlicher Weise kann auch ein Bodenteil 116 mit der zylindrischen Behälterwand 118 verklebt sein. Zum druckdichten Durchführen und Verkleben der einzelnen Rohranschlüsse 76, 78, 90, 92 sowie des Kondensatableitrohrs 42 durch die Bodenwand 116 sind entsprechend hochgezogene Bereiche 120 vorgesehen, die eine längere Klebefläche und damit eine größere Abdichtsicherheit ergeben. In ähnlicher Weise sind auch Befestigungsstücke 122 in den Behälterboden 116 eingeklebt. Diese weisen Befestigungsgewindelöcher 124 auf. Zur Erfassung der Soletemperatur kann der bereits erwähnte Öffnung 112 im Deckel 74 in den Behälter 38 eingeführt werden.

Zur Abstützung der Rohrleitung 62 können entsprechend Stützeinrichtungen 126 dienen, die von der Bodenplatte 116 bis zur Deckelplatte 74 reichen und ebenfalls vorzugsweise im Abstand von 120° zueinander dreifach vorgesehen sind.

Das Gehäuse 38 ist von einer Wärmeisolierung 128 umgeben, beispielsweise gebildet durch aufgeklebte bzw. übergeschobene Hartschaumstücke in Zylinderform (Bezugszahl 130) oder Plattenform (132, 134, 136 oder 138).

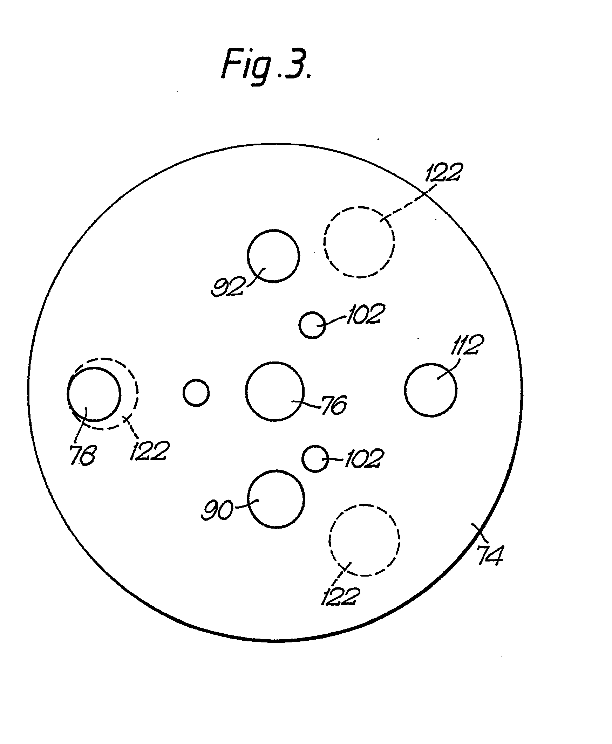

Fig. 3 zeigt eine Ansicht von oben auf den Behälter 38, insbesondere also den Deckel 74 mit den Anschlußrohren 76, 78 für das Meßgas sowie (in Fig. 2 versetzt gezeichnet) Anschlußrohren 90, 92 für das Kältemittel. Außerdem ist die Sensoröffnung 112 zu erkennen, des weiteren die Fußstücke 122 sowie die Nasen 102. Die auf der rechten Hälfte der Fig. 3 dargestellten Füsse 122 sowie die dazu ausgerichteten Nasen 102 sind in der Fig. 2 ebenfalls versetzt dargestellt.

## Patentansprüche

1. Meßgaskühleinrichtung (10), bestehend aus einem einen Teil des Kältemittelkreislaufs eines Kühlaggregats bildenden Wärmetauscher (30, 40), der zusammen mit einem Kondensatabscheider (18) in einem nach außen wärmeisolierten (128) Behälter (38) angeordnet ist, wobei der Wärmetauscher von einem in dem Behälter (38) im wesentlichen senkrecht angeordneten Rohrstück (56) gebildet wird, an dessen unterem Ende (58) eine Rohrleitung (62) mit einem Rohrdurchmesser (68) der kleiner als der des Rohrstücks (56) ist, angeschlossen ist, die um den Außenumfang (64) des Rohrstücks (56) wendelförmig mit nach oben gerichteter Wendelsteigung (66) gewickelt ist, und wobei das obere Ende (82) des Rohrstücks (56) und das obere Ende der Rohrleitungswendel (62) durch die Wand (74) des Behälters (38) dicht hindurchgeführt sind, dadurch gekennzeichnet, daß um das Rohrstück (56) eine zweite Rohrleitung (84) wendelförmig gewickelt ist, deren zwei Enden (86, 88) durch die Wand (74) des Behälters (38) dicht hindurchgeführt sind, und die über im Behälter (38) befindliche Wärmeübertragungsflüssigkeit (108) mit der ersten Rohrleitung (62) in Wärmekontakt steht und von Kältemittel des Kühlaggregats durchströmt ist, daß die zweite Rohrleitungswendel (84) in einer zwischen Rohrstückaußenfläche (64) und erster Rohrleitungswendel (62) gebildeten Ringraum (80) angeordnet ist, und daß die zweite Rohrleitungswendel (84) einen ersten äußeren Wendelteil (94) und einen zweiten, dazu koaxialen inneren Wendelteil (96) aufweist, wobei die Wendelteile (94, 96) an ihrem unteren Ende miteinander verbunden sind und an ihrem oberen Ende den Kältemitteleinlaß bzw. den Kältemittelauslaß bilden.

2. Meßgaskühleinrichtung nach Anspruch 1, dadurch gekennzeichnet, daß das obere Ende (86) der inneren Wendel (96) den Kältemitteleinlaß bildet.

3. Meßgaskühleinrichtung nach einem der Ansprüche 1 oder 2, dadurch gekennzeichnet, daß die zweite Rohrleitungswendel (84) von einer zylindrischen Wand (98) oder von zwei oder mehr Haltewinkeln (104) umschlossen bzw. gestützt ist.

4. Meßgaskühleinrichtung nach einem der Ansprüche 1 bis 3, dadurch gekennzeichnet, daß der Behälter (38) eine verschließbare Öffnung zum Einführen von Wärmeübertragungsflüssigkeit (108) wie Salzsole oder ein Gemisch aus Wasser und Ethandiol (Glykol), enthält.

5. Meßgaskühleinrichtung nach einem der Ansprüche 1 bis 4, dadurch gekennzeichnet, daß gasführende Teile aus säurefestem Kunststoff, wie PVDF (Polyvinylidenfluorid) bestehen.

6. Meßgaskühleinrichtung nach einem der Ansprüche 1 bis 5, dadurch gekennzeichnet, daß die Gehäuseteile (außer Kältemittel führenden Teilen) und/oder nicht gasführende Teile aus Kunststoff, wie PVC (Polyvinylchlorid) bestehen.

7. Meßgaskühleinrichtung nach Anspruch 5 oder 6, dadurch gekennzeichnet, daß die Verbindungen zwischen den Kunststoffteilen verklebt sind.

8. Meßgaskühleinrichtung nach Anspruch 5, 6 oder 7, dadurch gekennzeichnet, daß im Gehäuseboden (116) Befestigungsstücke (122) eingeklebt sind.

9. Meßgaskühleinrichtung nach Anspruch 5, 6, 7 oder 8, dadurch gekennzeichnet, daß am Gehäusedeckel (74) Nasen (102) zur Aufnahme von Befestigungsschrauben angespritzt sind.

10. Meßgaskühleinrichtung nach einem der Ansprüche 1 bis 9, dadurch gekennzeichnet, daß der Behälter (38) einen in die Kühlsole eingetauchten Sensor (50) aufweist.

11. Meßgaskühleinrichtung nach einem der Ansprüche , bis 4, dadurch gekennzeichnet, daß die Meßgas führenden Teile aus Glasrohr gebildet sind.

12. Meßgaskühleinrichtung nach einem der Ansprüche 1 bis 4, dadurch gekennzeichnet, daß die Meßgas führenden Teile aus hoch korrosionsfestem Stahl bestehen.

## Claims

1. Cooling device (10) for a gas to be measured, consisting of a heat exchanger (30, 40) which forms part of the refrigerant circuit of a cooling unit and which, together with a condensate separator (18), is arranged in a container (38) which is heat-insulated outwardly at (128), the heat exchanger being formed by a tube section (56) which is arranged substantially perpendicularly in the container (38) and to the bottom end (58) of which is connected a pipe conduit (62) with a pipe diameter (68) which is smaller than that of the tube section (56), which conduit is coiled around the external circumference (64) of the tube section (56) with an upwardly directed slope of the coil (66), the upper end (82) of the tube section (56) and the upper end of pipe conduit coil (62) being tightly conducted through the wall (74) of the container (38), characterised in that the pipe section (56) has helically wound around it a second pipe conduit (84), of which the two ends (86, 88) are led tightly through the wall (74) of the container (38), and which, through heat-transfer liquid (108) disposed in the container (38), has heat contact with the first pipe conduit (62) and has flowing therethrough refrigerant of the cooling assembly, that the second pipe conduit coil (84) is arranged in an annular space (80) formed between the outside of the pipe section (64) and first pipe conduit coil (62), and that the second pipe conduit coil (84) comprises a first outer coil section (94) and a second inner coil section (96) which is coaxial therewith, the coil sections (94, 96) being connected to one another at their bottom end and forming the refrigerant inlet and refrigerant outlet, respectively, at their upper end.

2. Cooling device for a gas to be measured, according to claim 1, characterised in that the upper end (86) of the inner coil (96) forms the refrigerant inlet.

3. Cooling device for a gas to be measured, according to one of the claims 1 or 2, characterised in that the second pipe conduit coil (84) is surrounded by a cylindrical wall or respectively is supported by two or more supporting angle

4. Cooling device for a gas to be measured, according to one of the claims 1 to 3, characterised in that the container (38) contains a closable opening for the introduction of heat transfer liquid (108), such as salt brine or a mixture of water and ethanediol (glycol).

5. Cooling device for a gas to be measured, according to one of the claims 1 to 4, characterised in that gas-conducting parts consist of an acid-resistant synthetic plastic, such as PVDF (polyvinylidene fluoride).

6. Cooling device for a gas to be measured, according to one of the claims 1 to 5, characterised in that the housing parts (except parts carrying refrigerant) and/or parts not carrying gas, consist of synthetic plastics, such as PVC (polyvinyl chloride).

7. Cooling device for a gas to be measured, according to claim 5 or 6, characterised in that the connections between the synthetic plastic parts are by adhesive.

8. Cooling device for a gas to be measured, according to claim 5, 6 or 7, characterised in that fixing elements (122) are stuck in the bottom (116) of the housing.

9. Cooling device for a gas to be measured, according to claim 5, 6, 7 or 8, characterised in that the housing cover (74) has lugs or projections (102) moulded thereon for accommodating fixing screws.

10. Cooling device for a gas to be measured, according to one of the claims 1 to 9, characterised in that the container (38) comprises a sensor (50) introduced into the cooling brine.

11. Cooling device for a gas to be measured, according to one of the claims 1 to 4, characterised in that the components carrying gas to be measured are formed of glass tubing.

12. Cooling device for a gas to be measured, according to one of the claims 1 to 4, characterised in that the components carrying said gas consist of steel with high corrosion-resistance.

## Revendications

1. Dispositif de refroidissement de gaz de mesure (10) constitué par un échangeur de chaleur (30, 40) qui forme une partie du circuit de réfrigérant d'un dispositif réfrigérant et qui est agencé avec un séparateur de condensat (18) dans un récipient (38) calorifugé vers l'extérieur (128), l'échangeur de chaleur étant constitué par un bout tubulaire (56) disposé essentiellement verticalement dans le récipient et raccordé à son extrémité inférieure (58) à un conduit (62) qui présente un diamètre de tube (68) plus petit que celui du bout tubulaire (56) et qui est enroulé autour du périmètre extérieur (64) du bout tubulaire (56) à la manière d'une hélice à pas orienté vers le haut, l'extrémité supérieure (82) du bout tubulaire (56) et l'extrémité supérieure du conduit en hélice (62) traversant de façon étanche la paroi (74) du récipient (38), caractérisé en ce qu'un deuxième conduit (84) est enroulé à la manière d'une hélice autour du bout tubulaire (56), conduit (84) dont les deux extrémités (86, 88) traversent de façon étanchée la paroi (74) du récipient (38) et qui est en contact thermique avec le premier conduit (62) via le liquide de transfert thermique contenu dans le récipient, ledit deuxième conduit (84) étant parcouru par un réfrigérant du dispositif réfrigérant, en ce que le deuxième conduit hélicoïdal (84) est agencé dans un espace annulaire (80) compris entre la surface extérieure du bout tubulaire (64) et le premier conduit hélicoïdal (62) et en ce que le deuxième conduit hélicoïdal (84) présente une première partie hélicoïdal extérieure (94) et une deuxième partie hélicoïdale intérieure (96) coaxiale par rapport à la première partie hélicoïdale, les parties hélicoïdales (94, 96) étant reliées entre elles à leur extrémité inférieure et constituant à leur extrémité supérieure l'admission ou la sortie de réfrigérant.

2. Dispositif de refroidissement de gaz de mesure selon la revendication 1 caractérisé en ce que l'extrémité supérieure (86) de l'hélice interne (96) constitue l'admission de réfrigérant.

3. Dispositif de refroidissement selon la revendication 1 ou 2 caractérisé en ce que la deuxième hélice (84) est enveloppée par une paroi cylindrique (98) ou supportée par deux ou plusieurs angles de retenue (104).

4. Dispositif de refroidissement selon l'une quelconque des revendications 1 à 3 caractérisé en ce que le récipient (38) comporte une ouverture obturable pour l'alimentation de liquide de transfert thermique (108) tel qu'une saumure ou un liquide constitué d'eau et d'éthanediol (glycol).

5. Dispositif de refroidissement de gaz de mesure selon l'une quelconque des revendications 1 à 4 caractérisé en ce que les parties transportant du gaz sont constituées par un matériau synthétique résistant à l'acide, comme le PVDF (polyfluorure de vinylidène).

6. Dispositif de refroidissement de gaz de mesure selon l'une quelconque des revendications 1 à 5 caractérisé en ce que les parties de boîtier (à part les parties transportant le réfrigérant) et/ou les parties ne transportant pas de gaz sont constituées par un matériau synthétique comme le PVC (polychlorure de vinyle).

7. Dispositif de refroidissement de gaz de mesure selon la revendication 5 ou 6 caractérisé en ce que les assemblages entre les éléments en matériau synthétique sont collés.

8. Dispositif de refroidissement de gaz de mesure selon la revendication 5, 6 ou 7 caractérisé en ce que des éléments de fixation (122) sont collés dans le fond de boîtier.

9. Dispositif de refroidissement de gaz de mesure selon la revendication 5, 6, 7 ou 8 caractérisé en ce que des ergots (109) pour le logement de vis de fixation sont injectés d'une pièce avec le couvercle de boîtier (74)

10. Dispositif de refroidissement de gaz de mesure selon l'une quelconque des revendications 1 à 9 caractérisé en ce que le récipient (38) présente une sonde (50) noyée dans la saumure de refroidissement.

11. Dispositif de refroidissement de gaz de mesure selon l'une quelconque des revendications 1 à 4 caractérisé en ce que les parties transportant du gaz de mesure consistent en tubes de verre.

12. Dispositif de refroidissement de gaz de mesure selon l'une quelconque des revendications 1 à 4 caractérisé en ce que les parties transportant du gaz de mesure consistent en acier très résistant à la corrosion.

Fig.1.

EP 0 308 531 B1

# Fig.2.

# Fig.3.